# EUROPEAN PATENT SPECIFICATION

(11) **EP 1 501 527 B1**
(45) Date of publication and mention of the grant of the patent: **02.09.2009**
(21) Application number: 03731077.8
(22) Date of filing: 01.05.2003
(51) Int. Cl.: A61K 36/22, A61P 31/04

(54) **USE OF MASTIC AND ITS ESSENTIAL OILS FOR THE CONTROL OF MICROBIAL INFECTIONS**
VERWENDUNG VON MASTIX UND DESSEN ÄTHERISCHEN ÖLEN ZUR VERHINDERUNG ODER BEKÄMPFUNG VON MIKROBIELLER INFEKTIONEN
UTILISATION DU MASTIC ET DE SES HUILES ESSENTIELLES DANS LE CONTROLE DES INFECTIONS MICROBIENNES

(30) Priority: 01.05.2002 US 376790 P; 18.09.2002 US 411665 P
(43) Date of publication of application: 02.02.2005
(73) Proprietor: LAVIPHARM S.A., 190 02 Peania Attica (GR)
(72) Inventor: FOTINOS, Spiros, Kolonaki, GR-106 72 Athens (GR); PANAITESCU, Ligia, Ano Dasos Chaidariou, GR-124 61 Attiki (GR); KLETSAS, Dimitris, Filothei, GR-152 37 Attiki (GR); PLATSINIS, Harris, GR-114 73 Athens (GR); ZERVOLEA, Irene, Glyfada, GR-166 74 Attiki (GR); SKALTSOUNIS, Alexios-Leandros, GR-151 27 Athens (GR); MITAKOU, Sofia, GR-151 27 Athens (GR); MAGIATIS, Prokopios, GR-Salamina (GR)
(74) Representative: Brown, David Leslie
(86) International application number: PCT/US2003/013728
(87) International publication number: WO 2003/092712

(56) References cited:
- EP-A- 1 178 104
- WO-A-01/21212
- GR-B- 1 003 541
- GR-B- 1 003 832
- GR-B- 1 003 868
- PATENT ABSTRACTS OF JAPAN vol. 2000, no. 22, 9 March 2001 (2001-03-09) & JP 2001 128643 A (NIHON YAKUGYO:KK), 15 May 2001 (2001-05-15)
- MAGIATIS PROKOPIOS ET AL: "Chemical composition and antimicrobial activity of the essential oils of Pistacia lentiscus var. chia." PLANTA MEDICA., vol. 65, no. 8, December 1999 (1999-12), pages 749-752, XP009017040 ISSN: 0032-0943
- IAUK L ET AL: "In vitro antimicrobial activity of Pistacia lentiscus L. extracts: Preliminary report." JOURNAL OF CHEMOTHERAPY, vol. 8, no. 3, 1996, pages 207-209, XP009017043 ISSN: 1120-009X
- TASSOU CHRYSOULA C ET AL: "Antimicrobial activity of the essential oil of mastic gum (pistacia lentiscus var. chia) on gram positive and gram negative bacteria in broth and in model food system" INT BIODETERIOR BIODEGRAD;INTERNATIONAL BIODETERIORATION BIODEGRADATION OCT-DEC 1995 ELSEVIER SCIENCE LTD, OXFORD, ENGL, vol. 36, no. 3-4, October 1995 (1995-10), pages 411-420, XP001154562
- TAKAHASHI KEISO ET AL: "A pilot study on antiplaque effects of mastic chewing gum in the oral cavity." JOURNAL OF PERIODONTOLOGY. UNITED STATES APR 2003, vol. 74, no. 4, April 2003 (2003-04), pages 501-505, XP009017045 ISSN: 0022-3492
- RAMAN A ET AL: "Antimicrobial effects of tea-tree oil and its major components on Staphylococcus aureus, Staph. epidermidis and Propionibacterium acnes" LETTERS IN APPLIED MICROBIOLOGY, OXFORD, GB, vol. 21, 1995, pages 242-245, XP002190828
- PAPAGEORGIOU V P ET AL: "Gas chromatographic-mass spectroscopic analysis of the acidic triterpenic fraction of mastic gum" JOURNAL OF CHROMATOGRAPHY A, ELSEVIER SCIENCE, NL, vol. 769, no. 2, 9 May 1997 (1997-05-09), pages 263-273, XP004064185 ISSN: 0021-9673

## Description

### Technical Field and Background Art

The present invention relates to pharmaceutical formulations for use in the treatment of microbial infections and cell hyperproliferation conditions. More particularly, the present invention relates to using the newly discovered anti-microbial and anti-cell hyperproliferation properties of certain natural extracts and isolates, or synthetic equivalents thereof, for treating microbial infection such as *H. Pylori* infection or for treating cell hyperproliferation conditions such as dandruff.

### Background of the Invention

As a means for establishing a competitive advantage in a competitive microbial world, microorganisms produce and excrete antimicrobial compounds that suppress the growth of, and even kill, other microorganisms. Other organisms also produce compounds that have antimicrobial properties. Humans have exploited this phenomenon in the treatment of infectious disease by identifying natural products from microbes, plants, and marine lifeforms that are successful against a variety of infectious diseases, or by developing synthetic varieties of similar compounds. Hundreds of such compounds are now known and used to treat bacterial and fungal infections, and more continue to be identified and developed as strains of microorganisms arise that are resistant to existing antimicrobial compounds.

Because of undesirable side-effects and sensitivity which can accompany the administration of microbial-produced and synthetic antimicrobial compounds, the use of agents derived from plants in the treatment of microbial diseases is of particular interest, both as a means for avoiding the side-effects and sensitivities, and as a source for additional anti-microbial compounds that may be effective in treating resistant strains of microbes.

One such agent is mastic resin (gum) derived from the plant *Pistacia lentiscus* var. *chia*, known since antiquity to have therapeutic effects. Genesis 37:25 refers to its use as a styptic substance (e.g. haemostatic agent), and Mediterranean countries have used it for centuries as a traditional remedy for the relief of abdominal pain, gastralgia, dyspepsia and peptic ulcer. However, renewed interest in the therapeutic effects of mastic gum has arisen, particularly the use of mastic gum to counter various pathological conditions caused by microbes.

For example, recent evidence suggests that administration of 500 mg/kg of mastic gum had a beneficial effect on the gastric epithelium of mice with gastric and duodenal ulcers (J. Ethnopharmacol, 15:271-278, 1986), and doses of 1g/day in humans with peptic ulcers displayed a therapeutic effect (Clin Exp Pharmacol Physiol 11:541-544; and N Engl J Med 339:1946, 1998). Therefore, understanding these results and investigating the antimicrobial and related therapeutic properties of mastic gum is desirable. With such knowledge, safer, more effective, and less costly treatments for preventing and controlling physiological and/or pathological conditions can therefore be developed using mastic gum and its components.

### Summary of the Invention

The present invention is directed to an oral or topical pharmaceutical formulation comprising a mastic extract product and a mastic essential oil, isolated from the species *Pistacia lentiscus* var. *chia*, for use in anti-microbial treatment of a subject..

In accordance with embodiments of the present invention, anti-microbial and anti-cell proliferation pharmaceutical formulations comprising one or more products isolatedfrom mastic resin, including whole extract, acid extract and neutral extract combined with essential oil or synthetic equivalents thereof, or comprising isolated component products or synthetic equivalents thereof, are described. Specifically, such pharmaceutical formulations may be used to treat microbial infections, including *H. pylori, Propionibacterium, Staphylococcus, Pseudomonas*, and cell hyperproliferation such as dandruff or gastrointestinal cancer. The formulations include oral and topical formulations. Such oral and topical formulations such as tablet, patch, cream, ointment, solution, gel, hydrogel, tincture, shampoo, and film, formulations of the mastic extract, essential oil, and isolated component products or synthetic equivalents thereof.

Methods for treating microbial infections in a subject so infected or at risk for such infection are also described. The methods include providing an anti-microbial agent comprising a mastic whole extract, acid extract, essential oil, isolated component or synthetic equivalent thereof in an oral or topical formulation, orally or topically administering an effective amount of the anti-microbial agent so as to treat the microbial infection. Oral administration may be carried out by administration of a tablet formulation, and topical administration may be carried out by administration of any appropriate topical formulation including a cream, ointment, solution, lotion, tincture, shampoo, patch, film, hydrogel or gel formulation.

Also described are methods for treating cell hyperproliferative conditions in a subject clinically manifesting cell hyperproliferation or a subject at risk for clinically manifesting cell hyperproliferation. The method comprises providing am anti-cell hyperproliferation agent comprising a mastic whole extract, acid extract, essential oil, isolated component or synthetic equivalent thereof in an oral or topical formulation, and topically administering an effective amount of the cell hyperproliferation agent so as to treat the cell hyperproliferation condition. Topical administration may be carried out by administration of any appropriate topical formulation including a cream, ointment, solution, lotion, tincture, shampoo, patch, film, hydrogel or gel formulation. Other embodiments include methods for treating the cell proliferation condition of dandruff by topically administering the anti-cell hyperproliferation agent once or twice daily as a shampoo formulation.

Also described are methods of manufacture of a pharmaceutical formulations for treatment of *H. pylori, Propionibacterium, Staphylococcus, Pseudomonas*, or proliferation. The methods include adding a mastic whole extract, acid extract, neutral extract, isolated component or synthetic equivalent thereof in an oral or topical formulation wherein the oral or topical formulation of the particular agent is used in the treatment of a particular infection or condition. For example, one particular embodiment provides a method of manufacture of a formulation of an anti-*H. pylori* agent, in which a mastic whole extract, acid extract, neutral extract, isolated component or synthetic equivalent thereof is added into an oral formulation wherein the oral formulation of the anti-*H. pylori* agent is used in the treatment of the *H. pylori* infection.

### Brief Description of the Drawings

The foregoing features of the invention will be more readily understood by reference to the following detailed description, taken with reference to the accompanying drawings, in which:

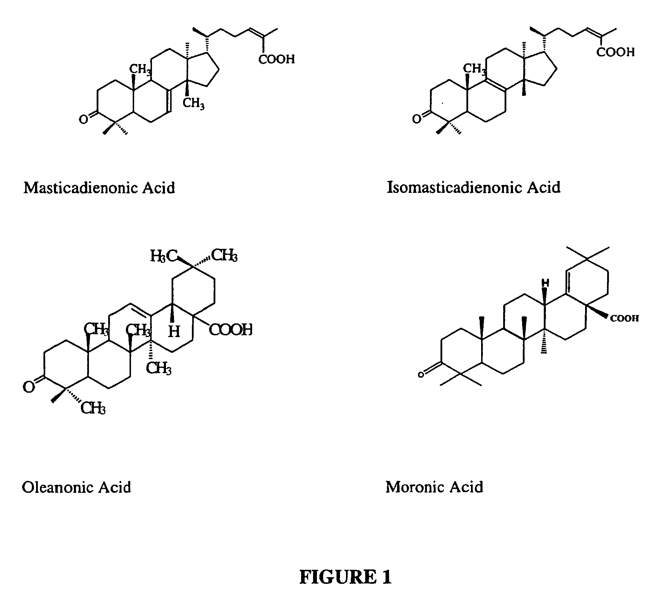

Fig. 1 shows examples of some major components from an acidic mastic fraction.
Fig. 2 shows the inhibition of human epidermoid carcinoma cell line A431 upon 48-hr treatments of varying concentrations of whole extract product, acidified extract product, and neutral extract product.

### Detailed Description of Specific Embodiments

Definitions. As used in this description and the accompanying claims, the following terms shall have the meanings indicated, unless the context otherwise requires:
"Plant product" as used herein means, any compound, material, component, substance, molecule, mixture, fraction, or ingredient derived from a plant source, whether physically obtained from the plant source or produced as a synthetic equivalent, including pharmaceutical preparations made with such plant products, wherein the plant product is the active ingredient in the pharmaceutical preparations.
"Mastic" as used herein means, the exudation (resin) derived from wounding the trunk of the bush *Pistacia lentiscus* var. *chia* of the family *Anadarciacea*, and the components therein, whether physically obtained from the actual plant resin or produced as a synthetic equivalent.
"Whole extract" as used herein means, any active fraction of mastic derived after a first solvent extraction of the resin.
"Acidic mastic fraction" as used herein means, any active acidic fraction of mastic derived from a whole extract.
"Neutral mastic fraction" as used herein means, any active neutral fraction of mastic derived from a whole extract.
"Essential mastic fraction" as used herein means, any active fraction of mastic including volatile components derived from a whole extract.
"Isolated component" as used herein means, any individual molecule, isolated from a mastic gum, a mastic extract, or a mastic essential oil.
"Microbes/microorganisms" as used herein mean, any autonomous or non-cellular formations that are pathogens or potential pathogens for humans.
"Anti-microbial activity" as used herein means, any ability of an agent or mixture of agents to inhibit or suspend growth of and/or destroy microbes/microorganisms.
"Anti-microbial agent" as used herein means, any extract, essential oil or isolated component from mastic or synthetic equivalent that is effective in preventing, reducing or controlling microbial infections.
"Anti-*H. pylori* agent" as used herein means, any extract, essential oil or isolated component from mastic or synthetic equivalent that is effective in preventing, reducing or controlling *H. pylori* infections.
"Anti-*Propionibacterium* agent" as used herein means, any extract, essential oil or isolated component from mastic or synthetic equivalent that is effective in preventing, reducing or controlling *Propionibacterium* infections.
"Anti-*Staphylococcus* agent" as used herein means, any extract, essential oil or isolated component from mastic or synthetic equivalent that is effective in preventing, reducing or controlling *Staphylococcus* infections.
"Anti-*Pseudomonas* agent" as used herein means, any extract, essential oil or isolated component from mastic or synthetic equivalent that is effective in preventing, reducing or controlling *Pseudomonas* infections.
"Anti-Cell hyperproliferation agent" as used herein means, any extract, essential oil or isolated component from mastic or synthetic equivalent that is effective in preventing, reducing or controlling cell hyperproliferation.
"Anti-dandruff agent" as used herein means, any extract, essential oil or isolated component from mastic or synthetic equivalent that is effective in preventing, reducing or controlling dandruff.

Abnormal microbial colonization of cells in a living subject and sometimes-associated cell hyperproliferative disorders are the hallmark of a number of diseases and conditions that affect subjects where subjects are for example, animals, for example, mammals, for example humans. These diseases include epidermoid carcinoma, colon adenocarcinoma, mammary adenocarcinoma, or lung carcinoma, and *Helicobacter* associated gastrointestinal cancer, *Helicobacter*-induced gastritis, gastric ulcers, chronic tonsillitis, mucosa-associated and chronic pulmonary infections caused by bacteria susceptible to the microbe inhibitory effects of the *Pistacia lentiscus* mastic gum and isolates derived therefrom or their synthetic counterparts. Other diseases include skin diseases such as psoriasis and associated dandruff, keloid scarring, pre-cancerous skin growths such as actinic keratoses, vascular diseases including diabetic retinopathy associated with excess growth of blood vessels as well as smooth muscle proliferation associated with arteriosclerotic plaque and restenosis and cancer which can afflict any organ or tissue in the body. These diseases should be preventable and/or treatable with cytostatic or cytotoxic agents.

It may be desirable to deliver the active agents specifically to target cells. This can be achieved using cell specific carriers such as tissue specific antibody, other protein, lipid or polysaccharide having specificity for a ligand on the cell surface molecule or within a cell.

We have identified a novel class of compounds having cytostatic or cytotoxic activity for use in one embodiment for treating cell hyperproliferative diseases. This class of compounds is characterized as having a chemical composition that is substantially equivalent to at least one composition obtained from and characteristic of the plant family of *Anacardiaceae*. An example of this plant family is *Pistacia lentiscus, Pistacia lentiscus* var. *chia*. These plants are native of the island of Chios.

In one embodiment of the invention, agents active against cell hyperproliferation are obtained by chemical processes examples of which are provided below in Example 1, using an anti-cell hyperproliferation assay such as that described in Example 6.

In another embodiment, the class of compounds has anti-microbial activity useful for treating persistent microbial infections such as *H. pylori* infection of the gastrointestinal tract, thereby providing effective treatment for gastric ulcers and ulcerative conditions, as well as preventing or inhibiting hyperproliferative conditions exacerbated or initiated in-part by persistent microbial infections of mucosal tissues.

In one embodiment of the invention, such anti-microbial agents are obtained by chemical processes examples of which are provided below in Example 1 using an anti-microbial assay such as described in Examples 2A and 3.

The first extract (total fraction or whole extract) contains all the compounds of the mastic gum except the polymer resin. The second extract (acid fraction) contains all the triterpenic acids of the total fraction including oleanonic acid, moronic acid, masticadienonic acid and isomasticadienonic acid. The third extract (neutral fraction) contains all the other terpenes of the total fraction except the triterpenic acids. The major constituents of the third extract include tirucallol, oleanolic aldehyde, and betulonal.

Embodiments of the present invention take advantage of the discovery that *Pistacia lentiscus* produces a mastic gum that contains constituents with cytotoxic or cytostatic activity with respect to a variety of cancer cell lines. Three extracts derived from *Pistacia lentiscus* var. *chia* have been tested for their cytotoxic/cytostatic activity against four established cancer cell lines, i.e. human mammary adenocarcinoma MCF-7, human colon adenocarcinoma HT-29, human lung carcinoma A549, and human epidermoid carcinoma A431. The results indicated that all three extracts possess a considerable inhibitory activity against the four cancer cell lines, with IC₅₀ in the range of 10 to 100 µg/mL. (see Examples 6-8 and Table 9-11)

The active agent has been prepared for *in vitro* experimentation as described in Examples 6-8. The examples show that mastic essential oil is useful in concentrations of between 0.3-3 µg/mL of culture medium. An effective dose of mastic essential oil in a formulation provides significant inhibitory behavior against all cancerous cell lines examined including epidermoid carcinoma, colon adenocarcinoma and lung carcinoma.

The active agent may be incorporated into pharmaceutical formulation for administration to the subject as exemplified in Example 9 below. For example, any of the extracts from the mastic gum or the essential oil as well as any of the isolated major components oleanonic acid, moronic acid, isomasticadienonic acid and isomasticadienolic acid, among others, may be incorporated into pharmaceutical formulations for administration to a subject. For example, an effective daily dose of 1 to 5 g of mastic extract, essential oil or active component may be administered orally to humans. Particularly, oral formulations of tablets containing varying concentrations of active mastic extract, essential oil and/or active isolated components or synthetic equivalents are envisioned. The oral formulation may include about 1-63% of mastic extract or essential oil product or mastic isolated component as required. Mastic isolated components include masticadienonic acid, aldehyde or alcohol; masticadienolic acid, aldehyde or alcohol; isomasticadienonic acid aldehyde or alcohol; isomasticadienolic acid, aldehyde or alcohol; oleanonic acid, aldehyde or alcohol; moronic acid, aldehyde or alcohol; α-pinene; β-myrcene; tirucallol; betulonal, or synthetic equivalents thereof. However, an effective dose of the active ingredients for administration to a subject should be determined by an experienced medical practitioner based on the clinical profile of the patient and the route of administration. Considerations include the patient's size, weight, type and severity of the condition.

Examples 6 -8 show *in vitro* protocols and results for *in vitro* treatments of four human cancer cell lines with whole extract, acid extract, neutral extract and essential oil preparations of mastic.

Example 9 shows various example formulations for extract and isolated components from mastic.

Examples 10 shows *in vivo* results and protocols for *in vivo* treatments of *H. pylori-*infected mice with whole extract preparations of mastic.

Examples 11-14 show *in vivo* protocols for *in vivo* treatment of humans infected with *H. pylori* (Examples 11-12), and other bacteria (Examples 13-14) using mastic extract fractions, mastic essential oil, or isolated mastic components.

Examples 15-16 show *in vivo* protocols for *in vivo* treatment of humans manifesting cell hyperproliferation using mastic extract fractions, mastic essential oil, or mastic isolated components.

### Example 1A: Isolating components from the plant Pistacia lentiscus var. chia

The method for isolating components of *Pistacia lentiscus* var. *chia* is described generally below:

### A) Collecting the soluble components, removing the insoluble polymer

Raw mastic gum in the form of a powder from the species *Pistacia lentiscus* var. *chia* is extracted with a first polar organic solvent or mixture of polar organic solvents, for example an ether/methyl alcohol mixture, to obtain a whole extract as follows. Following extraction of the raw mastic powder with a first polar organic solvent or mixture of organic solvents, the supernatant is separated from the solid insoluble polymer precipitate by filtration. The polar solvent extraction is repeated on the precipitate to separate out any further supernatant. The two supernatants are mixed and subsequently concentrated resulting in a dry residue. The dry residue contains all the components of mastic that are soluble in organic solvents. This is referred to as the total fraction or whole extract.

### B) Separating the acidic and neutral components of the whole extract

The whole extract in the form of dry residue is dissolved in a second polar organic solvent or mixture of polar organic solvents, for example ether or ethyl acetate or a mixture thereof, and is then extracted with a 5% solution of Na₂CO₃. The organic phase, herein termed the first organic phase, includes the neutral fraction and contains di-tri- cyclic terpenes that do not bear the carboxyl group.

The aqueous phase together with the intermediate phase, herein termed the emulsion phase, are mixed and then acidified using HCl. The acidified mixed aqueous phase is then re-extracted with a third polar organic solvent or mixture, which may be the same as the first or second polar organic solvents or may be different, for example, the third organic solvent may be just ether, to obtain a second organic phase. The second organic phase is then evaporated to dryness to obtain an acidified fraction product. Next, the first organic phase, which includes the neutral fraction, is evaporated to dryness to obtain a neutral fraction product.

### C) Isolating the various components from the extract fractions

From any of the fractions obtained, the individual components can be further separated and isolated using column chromatography, as is described in Example 1B section C), below.

Examples of polar organic solvents which may be used as first, second and third organic solvents, and mixtures thereof, include methanol, ethanol, propanol, isopropanol, neopentanol, ethyleneglycol, diethyl ether, methylethyl ether, ethylpropyl ether, methylpropyl ether, ethyl acetate, methylacetate, acetamide, acetaldehyde, tetrahydrofuran, dihydrofuran, furan, methyl acetate, ethylformate, methylformate, ethylpropanate, methylpropanate, dichloromethane, dimethylsulfoxide, dimethylacetamide, n,n,dimethylformamide, acetone, acetonitrile, and other polar organic solvents that successfully extract mastic fractions with activity against *H. pyori* or other bacteria in an *in vitro* screening assay, such as described in Examples 2A-2C through Example 6.

A specific example of an extraction protocol is described below.

### Example 1B: Isolating components from the plant Pistacia lentiscus var. chia

### A) Separating the acidic and neutral components of the whole extract:

One kg of raw mastic gum in the form of powdered resin (M0) was extracted with an ether/methyl alcohol mixture (1:7 v/v). The supernatant was separated from the solid insoluble polymer precipitate which is subsequently separated out by filtration. The extraction was repeated on the precipitate to separate out any further supernatant. The two supernatants were mixed and subsequently concentrated resulting in a dry residue. The dry residue contained all the components of mastic that are soluble in organic solvents. This is referred to as the total fraction or whole extract (M2).

### B) Separating the acidic and neutral components of the whole extract:

The dry residue was dissolved in ether or ethyl acetate and was extracted with a 5% solution of Na₂CO₃. The organic phase, termed the first organic phase, includes the neutral fraction (M3) and contains di-tri- cyclic terpenes that do not bear the carboxyl group. This neutral first organic phase may then be evaporated to dryness to yield a neutral fraction product.

The aqueous phase together with the intermediate phase (emulsion phase) was acidified using HCl and re-extracted with ether to produce a second organic phase. The second organic phase, which is the acidic fraction (M4), may then be evaporated to dryness to yield an acidic fraction product.

### C) Isolating the various acidic components:

The acidic fraction, as derived from stage (B) was further separated into its components through column chromatography.

The acidic fraction of the mastic total extract was submitted to MPLC over silica gel (20-40 mesh). Elution with CH₂Cl₂ containing increasing amounts of MeOH afforded masticadienonic acid **(1),** isomasticadienonic acid **(2),** isomasticadienolic acid **(3),** oleanonic acid **(4),** and moronic acid **(5).** The fractionation gave the following concentrations by weight 25% masticadienonic acid, 25% isomasticadienonic acid, 10% oleanonic acid and 5% moronic acid. Chemical and physical characterization of each isolated component is listed below.
**Masticadienonic acid (1).** Yellowish powder. EIMS *m*/*z* 454 (M+). ¹H-NMR (CDCl₃), see table 1. ¹³C-NMR (CDCl₃), see table 2.
**Isomasticadienonic acid (2).** Yellowish powder. EIMS *m*/*z* 454 (M+). ¹H-NMR (CDCl₃), see table 1. ¹³C-NMR (CDCl₃), see table 2.
**Isomasticadienolic acid (3).** White powder. [α]_{D}: -21° (c 0.4, CHCl₃). EIMS *m*/*z* 456 (M+). ¹H-NMR (CDCl₃), see table 1. ¹³C-NMR (CDCl₃), see table 2.
**Oleanonic acid (4)**. White gum. EIMS *mlz* 454 (M+). ¹H-NMR (CDCl₃), see table 1. ¹³C-NMR (CDCl₃), see table 2.
**Moronic acid (5).** Colorless powder. [α]_{D}: +59.3° (c 1.01, CHCl₃). EIMS *mlz* 454 (M+). ¹H-NMR (CDCl₃), see table 1. ¹³C-NMR (CDCl₃), see table 2.

Chemical shift data for both proton and carbon-13 NMR are shown below in Tables 1 and 2, respectively.

**TABLE 1 ¹H-NMR chemical shifts for compounds 1-5 (see above)**

| **H** | **1** | **2** | **3** | **4** | **5** |
|---|---|---|---|---|---|
| 1 | | 1.93 m | | | |
| 2 | | 2.49 m | | | |
| 3 | | | 3.45 t (2.9) | | |
| 4 | | | | | |
| 5 | | 1.65 t | | | |
| 6 | | 0.98 m | | | |
| 7 | 5.30 dd (3.5, 4.5) | 2.02 m | | | |
| 8 | | | | | |
| 9 | | | | | |
| 10 | | | | | |
| 11 | | 1.92 m | | | |
| 12 | | 1.62 t | | 12.30 m | |
| 13 | | | | | |
| 14 | | | | | |
| 15 | | 1.18 t | | | |
| 16 | | 1.90 m | | | |
| 17 | | 1.43 m | | | |
| 18 | 0.82 s | 0.69 s | 0.78 s | | |
| 19 | 1.12 s | 0.98 s | 0.97 s | | 5.10 s |
| 20 | | 1.00 m | | | |
| 21 | 0.90 d (5.0) | 0.85 d | 0.93 d (6.2) | | |
| 22 | | 1.55 m | | | |
| 23 | | 2.40 m | | 1.13 s | 1.02 s |
| 24 | 6.73 dt (8.0, 1.5) | 6.01 t | 6.09 tq (7.5, 1.4) | 1.07 s | 0.96 s |
| 25 | | | | 0.89 s | 0.89 s |
| 26 | | | | 1.04 s | 0.96 s |
| 27 | 1.83 d (1.5) | 1.84 s | 1.92 d (1.4) | 0.78 s | 0.73 s |
| 28 | 1.00 s | 0.98 s | 0.98 s | | |
| 29 | 1.04 s | 1.02 s | 0.87 s | 1.02 s | 0.94 s |
| 30 | 1.00 s | 0.82 s | 0.88 s | 0.92 s | 0.91 s |

**TABLE 2 ¹³C-NMR chemical shifts for compounds 1-5 (see above)**

| **C** | **1** | **2** | **3** | **4** | **5** |
|---|---|---|---|---|---|
| 1 | 38,3 | 35,7 | 29,6 | 38,9 | 39,7 |
| 2 | 34,7 | 34,7 | 26,7 | 34,0 | 33,9 |
| 3 | 216,6 | 218,3 | 78,9 | 217,7 | 218,3 |
| 4 | 47,6 | 47,2 | 36,4 | 47,3 | 47,1 |
| 5 | 52,1 | 51,5 | 44,0 | 55,2 | 54,7 |
| 6 | 24,3 | 20,4 | 18,8 | 19,4 | 19,5 |
| 7 | 117,6 | 27,6 | 27,5 | 32,0 | 33,4 |
| 8 | 145,7 | 132,6 | 133,3 | 39,1 | 40,4 |
| 9 | 48,3 | 134,6 | 133,9 | 46,8 | 50,3 |
| 10 | 34,7 | 37,1 | 37,1 | 36,7 | 36,8 |
| 11 | 27,3 | 21,5 | 21,4 | 23,5 | 21,4 |
| 12 | 33,4 | 30,8 | 29,6 | 122,2 | 25,9 |
| 13 | 43,4 | 44,1 | 44,0 | 143,6 | 41,3 |
| 14 | 50,9 | 50,0 | 49,9 | 41,6 | 42,4 |
| 15 | 32,1 | 29,9 | 30,7 | 27,6 | 29,2 |
| 16 | 27,9 | 28,2 | 27,9 | 22,8 | 33,6 |
| 17 | 52,5 | 50,2 | 50,8 | 46,5 | 47,8 |
| 18 | 20,3 | 15,7 | 15,6 | 41,6 | 136,4 |
| 19 | 18,3 | 19,9 | 20,0 | 45,7 | 133,0 |
| 20 | 35,8 | 36,6 | 35,8 | 30,6 | 31,9 |
| 21 | 18,3 | 18,7 | 18,5 | 33,7 | 33,3 |
| 22 | 35,4 | 36,0 | 35,1 | 32,3 | 33,2 |
| 23 | 26,9 | 27,0 | 26,7 | 26,3 | 26,7 |
| 24 | 146,6 | 147,4 | 146,1 | 21,3 | 20,8 |
| 25 | 125,9 | 125,8 | 126,1 | 14,9 | 16,4 |
| 26 | 173,0 | 173,4 | 172,4 | 16,9 | 15,7 |
| 27 | 12,6 | 21,5 | 20,6 | 25,8 | 14,7 |
| 28 | 21,3 | 20,8 | 27,9 | 217,8 | 182,8 |
| 29 | 24,3 | 26,9 | 22,7 | 33,0 | 30,2 |
| 30 | 21,4 | 24,4 | 24,3 | 23,5 | 29,0 |

### D) Isolating mastic essential oil:

In accordance with an alternate method, mastic essential oil is isolated from the raw mastic gum. The mastic gum on being extracted from the *Pistacia lentiscus* var. *chia* bush was reduced to powder, mixed with water and distilled in order to acquire the essential oil (M1). In particular, 1 Kg of mastic gum powder was added to 3 liters of H₂O and the mixture was distilled in a Clevenger modified apparatus with a water-cooled oil receiver to reduce overheating artifacts. After 3 hours a total of 28 mL of essential oil was obtained. The essential oil was dried over anhydrous sodium sulphate and stored at 4-6°C. The oil was light yellow with optical rotation [a]_{D}: 38.7° (CHCl₃, c 0.86). The essential oil contains monoterpenes, for example α-pinene and β-myrcene, etc.

### Example 2A: In vitro H. pylori Inhibition Assay for Screening Active Mastic Fractions and Isolated Components

*H. pylori* strains from patients with gastritis, duodenal or gastric ulcer as described below, and analogously, *H. pylori* strains from patients with stomach or intestinal cancer, are re- cultured on Petri dishes. After 48-hour incubation under microaerobic conditions, as described below, colonies are transferred to 1 mL of Brain Heart infusion Broth - BHIB (Oxoid) with 10% Fetal Calf Serum - FCS (Flow Laboratories, Irvine, Scotland) in order to reach a suspension whose turbidity is comparable to that of number 0.5 of the McFarland standard scale. The suspensions are transferred to 9 mL of BHIB medium and subsequently incubated for a period of 18-24 hours in anaerobic conditions and under constant stirring at 35°C in order for the bacteria to achieve their logarithmic growth phase. Turbidity is then adjusted utilizing BHIB to correspond to number 0.5 of the McFarland standard turbidity scale (approximately 10^{g} -10⁹ CFU/mL). The suspensions form the standardized inocula that are required for each strain of *H. pylori,* whether the strain is from an individual with both *H. pylori* infections and also a) a gastric ulcer, b) a duodenal ulcer, c) gastritis, or d) stomach or intestinal cancer.

Following each extraction step in the mastic extraction protocol of Examples 1A and 1B, a series of dilutions of the extract to be assayed (whole extract, acidic fraction extract, neutral extract, emulsion phase, or essential oil) or isolated component is performed as described below. Then, 100 µL of each dilution is added to sterile liquid cultures. The concentrations for the dilution series range from approximately 0.025 to 2.0 mg/mL for all the mastic preparations and isolated components.

The liquid cultures containing various dilutions of mastic extract fractions or isolated components, and controls having no mastic extract fractions or isolated components, are incubated at 35°C for 24 hours, in microaerobic conditions under constant stirring. Bacterial growth is followed by turbidity measurements using optical density (OD) measurements. Decreased *H. pylori* growth relative to a control is used to follow active fractions or determine activity of isolated components.
Method Controls: In each instance the following controls are performed
· Purity Control of the initial culture
· Growth Control of the standardized microorganism suspension by measuring the OD
· Sterility control
· Control on the antimicrobial activity of the solvent (ethanol, ether, ethyl acetate, DMSO,etc, as the case may be)

### Example 2B: In vitro Propionibacterim Acnes Inhibition Assay for Screening Active Mastic Fractions and Isolated Components

*Propionibacterium acnes* strain CBIP 53117 is re-cultured on a blood-containing anaerobic culture medium [Brucella Broth (BBL) with Bacto Agar, 0.5% (Difco Laboratories, Detroit, MI, USA) and vitamin K, 1 µg/mL and haemine, 5 µg/mL (Sigma-Aldrich Ltd, St. Louis, MO, USA) and 5% horse blood]. After a 48-hour incubation under microaerobic conditions with stirring, 3-5 colonies are transferred to 5 mL of pre-reduced thioglycolic broth (Oxoid) in order to reach a suspension for each strain whose turbidity is comparable to that of number 0.5 of the McFarland standard scale. Then 0.1 mL of each suspension is transferred once more to 5 mL of MHB and incubated with stirring at 35°C for 3-6 hours under anaerobic or aerobic conditions, as needed, in order for the bacterium to achieve a logarithmic growth phase. Turbidity is then adjusted utilizing additional growth medium to correspond to number 0.5 of the McFarland standard turbidity scale (approximately 10⁸ - 10⁹ CFU/mL). This suspension forms the standardized inoculum that is required for each strain of bacteria to be assayed.

Following each extraction step in the mastic extraction protocol of Examples 1A and 1B, a series of dilutions of the extract to be assayed (whole extract, acidic fraction extract, neutral extract, emulsion phase, or essential oil) or isolated component is performed as described below and 100 µL of each dilution is added to sterile liquid cultures. The concentrations for the dilution series range from approximately 0.025 to 2.0 mg/mL for all the mastic preparations or isolated components.

The liquid cultures containing various dilutions of mastic extract fractions or isolated components, and controls having no mastic extract fractions or isolated components, are incubated at 35°C for 24 hours, in microaerobic or aerobic conditions, as required, under constant stirring. Bacterial growth is followed by turbidity measurements using optical density (OD) measurements. Decreased bacterial growth relative to a control is used to follow active fractions or determine activity of isolated components. Method Controls: In each instance the following controls are performed
· Purity Control of the initial culture
· Growth Control of the standardized microorganism suspension by measuring the OD
· Sterility control
· Control on the antimicrobial activity of the solvent (ethanol, ether, ethyl acetate, DMSO,etc, as required)

### Example 2C: General In vitro Bacterial Inhibition Assay for Screening Active Mastic Fractions and Isolated Components

*Staphylocaccus, Enterobacter, Eschirichia, Salmonella, Pseudomona, Klebsiella* and *Lactobacillus* strains are isolated from patients suffering from such infections, as was done with *H. pylori* strains, and cultured on solid media Trypticase Soya Agar (TSA (Oxoid). After 24-hour incubation, colonies are transferred to 5 mL of the Mueller-Hinton Broth (MHB -Oxoid) in order to reach a suspension for each strain whose turbidity is comparable to that of number 0.5 of the McFarland standard scale. Then 0.1 mL of each suspension is transferred once more to 5 mL of MHB and incubated with stirring at 35°C for 3-6 hours under anaerobic or aerobic conditions, as needed, in order for the bacterium to achieve a logarithmic growth phase. Turbidity is then adjusted utilizing 0.85% NaCl to correspond to number 0.5 of the McFarland standard turbidity scale (approximately10⁸ - 10⁹ CFU/mL). This suspension forms the standardized inoculum that is required for each strain of bacteria to be assayed.

Following each extraction step in the mastic extraction protocol of Examples 1A and 1B, a series of dilutions of the extract to be assayed (whole extract, acidic fraction extract, neutral extract, emulsion phase, or essential oil) or isolated components is performed as described below and 100 µL of each dilution is added to sterile liquid cultures. The concentrations for the dilution series range from approximately 0.025 to 2.0 mg/mL for all the mastic preparations or isolated components.

The liquid cultures containing various dilutions of mastic extract fractions or isolated components, and controls having no mastic extract fractions or isolated components, are incubated at 35°C for 24 hours under constant stirring. Bacterial growth is followed by turbidity measurements using optical density (OD) measurements. Decreased bacterial growth relative to a control is used to follow active fractions or determine activity of isolated components.
Method Controls: In each instance the following controls are performed
· Purity Control of the initial culture
· Growth Control of the standardized microorganism suspension by measuring the OD
· Sterility control
· Control on the antimicrobial activity of the solvent (ethanol, ether, ethyl acetate, DMSO,etc, as required

### Example 3: Determining the antibacterial activity of mastic preparations on strains of Helicobacter pylori

### Strain isolation:

The clinical strains of *H. pylori* utilized were isolated at the Bacteriology Laboratory of the Hellenic Pasteur Institute during the year 1999. The strains were isolated from biopsies of the gastric antrum that were taken from patients with gastritis, duodenal or gastric ulcer (codes LAVHP-1 - LAVHP-17). One strain was from CCUG Culture Collection.

The clinical strains were isolated on a selective medium, Wilkins-Wilkins anaerobe agar (CWA) (BBL, Becton Dickinson Microbial System, Cockeysville, MD, USA) containing 7% horse blood, 10% horse serum (biochrom, KG, Berlin, Germany), Vitox (Oxoid) and *Helicobacter pylori* Selective Supplement (Dent's) (Oxoid). The Petri plates were incubated at 37°C for up to 7 days under microaerobic conditions with the use of envelopes to create anaerobic conditions without catalyst in anaerobic condition flasks (GasPak anaerobic system, BBL, BD). The identification of the suspect colonies was completed by microscopic examination of the wet preparation, microscopic preparation by Gram staining and urease, catalase and oxidase reactions. The *H. pylori* strains were maintained at -70°C in Brain Heart Infusion Broth (Oxoid) with glycerol until use.

**TABLE 3 Details of H. pylori strains used in assaying mastic fractions for anti-microbial activity.**

| **STRAIN CODE** | **DATE OF ISOLATION** | **SEX OF PATIENT** | **AGE** | **DIAGNOSIS (*)** |
|---|---|---|---|---|
| LAVHP-1 | 17-3-99 | M | 64 | D.U. |
| LAVHP-2 | 17-3-99 | M | 32 | G.U. |
| LAVHP-3 | 30-3-99 | M | 45 | D.U. |
| LAVHP-4 | 4-4-99 | F | 34 | GASTRITIS |
| LAVHP-5 | 24-4-99 | F | 33 | D.W.U. |
| LAVHP-6 | 27-4-99 | M | 67 | GASTRITIS |
| LAVHP-7 | 3-5-99 | F | 35 | GASTRITIS |
| LAVHP-8 | 4-5-99 | M | 15 | GASTRITIS |
| LAVHP-9 | 4-5-99 | M | 45 | D.U. |
| LAVHP-10 | 10-5-99 | M | 43 | D.U. |
| LAVHP-11 | 11-5-99 | M | 71 | G.U. |
| LAVHP-12 | 15-5-99 | M | 55 | D.U. |
| LAVHP-13 | 16-5-99 | F | 40 | D.W.U. |
| LAVHP-14 | 2-6-99 | F | 56 | D.W.U. |
| LAVHP-15 | 12-6-99 | M | 64 | GASTRITIS |
| LAVHP-16 | 13-6-99 | M | 60 | GASTRITIS |
| LAVHP-17 | 13-6-99 | M | 55 | D.U. |
| CCUG 38771 | - | - | - | - |

| | | | | |
|---|---|---|---|---|
| (*) D.U. = duodenal ulcer G.U. = gastric ulcer D.W.U.= dyspepsia without ulcer | | | | |

In order to control the antibacterial activity of the mastic preparations the Minimal Bactericidal Concentration (MBC) was determined.
Principle of the method: The *H. pylori* strains were incubated in liquid growth medium in the presence of a dilution series of successively halved concentrations of each mastic preparation. Subsequently each dilution was re-cultivated on solid growth medium, suitable for the growth of *H. pylori.* The lowest concentration of the agent that caused the number of CFUs to fall by 99.9% in comparison to the initial inoculum was called the MBC. Preparing the strain inoculum: Each strain of *H. pylori* was re-cultivated from -70°C on solid growth medium, CWA. After 48-hour incubation under microaerobic conditions, as described hereinabove, colonies were transferred to 1 mL of Brain Heart infusion Broth - BHIB (Oxoid) with 10% Fetal Calf Serum - FCS (Flow Laboratories, Irvine, Scotland) in order to reach a suspension whose turbidity was comparable to that of number 0.5 of the McFarland standard scale. This suspension was transferred to 9 mL of BHIB medium and was subsequently incubated for a period of 18-24 hours in anaerobic conditions and under constant stirring at 35°C in order for the bacterium to achieve its logarithmic growth phase. Turbidity was then adjusted utilising BHIB to correspond to number 3 of the McFarland standard turbidity scale (approximately 10⁸ -10⁹ CFU/mL). This suspension formed the standardized inoculum that was required for each strain of *H. pylori.*
Preparing the agents under examination - and the microtitre trays: In order to determine the MBC, sterile microtitre trays with 96 wells apiece (Sarstedt, Numbrecht, Germany) were utilized. Into these wells a dilution series of halved dilutions of selected concentrations of the mastic preparations being examined were added. These concentrations were prepared by dilutions of the preparations in BHIB and each well received 100 µL of each dilution. The range of concentrations for the dilution series examined was 0.045, 0.09, 0.195, 0.39, 0.78 and 1.56 mg/mL for all the mastic preparations except for mastic preparation 4 in ethanol which was examined at the concentrations 0.06, 0.12, 0.24, 0.48, 0.96 and 1.92 mg/mL.
Inoculation and incubation: To each well in the microtitre tray containing a dilution of agent, 10 µL of the microorganism suspension were added. Subsequently, the microtitre trays were incubated at 35°C for 24 hours, in microaerobic conditions under constant stirring. In order to determine the MBC, following the 24-hour incubation of the microtitre trays the contents of each well were mixed by aspiration and redistribution. Subsequently 100 µL of the contents from each well were spread on the surface of a CWA plate. This was then incubated in microaerobic conditions at 35°C for 48 hours, followed by the reading of the results.
Method Controls: In each instance the following controls took place
1. Purity Control of the initial culture
2. Growth Control of the standardized microorganism suspension by measuring the CFU/mL.
3. Sterility control
4. Control on the antimicrobial activity of the solvent (ethanol, ether, ethyl acetate, DMSO,etc, as required)
Reading the results: Each reculturing plate was examined and the number of colonies that developed was noted. The plates that displayed up to 300 CFUs were mentioned. The lowest concentration of the mastic preparation that displayed 99.9% fewer CFUs compared to the initial standardised inoculum was defined as the MBC for that strain.

Table 4 displays the values of MBCs determined.

**TABLE 4: MBC (µg/ml) for mastic preparations against strains of H. pylori.**

| M0 = powdered resin; M1 = essential oil; M2 = whole extract; M3 = neutral extract; M4 = acid extract | | | | | | | | |
|---|---|---|---|---|---|---|---|---|
| **Mastic fraction** | ***Helicobacter pylori* Strain** | | | | | | | |
| | **LAV** **HP-1** | **LAV** **HP-2** | **LAV** **HP-3** | **LAV** **HP-4** | **LAV** **HP-5** | **LAV** **HP-6** | **CCUG** **38771** | **LAV** **HP-7** |
| M0-EtOH | 0.09 | 0.195 | N/A | 0.195 | 0.195 | 0.195 | 0.39 | 0.195 |
| M0-DMSO | 0.195 | 0.09 | N/A | 0.195 | 0.195 | 0.195 | 0.195 | 0.195 |
| M1-EtOH | 0.39 | 0.39 | N/A | 0.195 | 0.195 | 0.39 | 0.78 | 0.195 |
| M1-DMSO | N/A | 0.195 | 0.39 | 0.195 | 0.09 | 0.195 | 1.56 | 0.195 |
| M2-EtOH | N/A | N/A | 0.195 | 0.195 | 0.195 | 0.39 | 0.39 | 0.195 |
| M2-DMSO | N/A | N/A | N/A | 0.195 | 0.39 | 0.195 | 0.78 | 0.195 |
| M3-EtOH | N/A | N/A | 0.39 | 0.78 | 0.78 | 0.78 | 1.58 | 0.39 |
| M3-DMSO | N/A | 0.39 | 0.78 | 0.39 | 0.195 | 0.78 | 1.56 | 0.39 |
| M4-EtOH | N/A | N/A | N/A | 0.12 | 0.12 | 0.12 | 0.24 | 0.12 |
| M4-DMSO | N/A | N/A | N/A | 0.195 | 0.39 | 0.195 | 0.78 | 0.195 |
| | | | | | | | | |

| **Mastic fraction** | ***Helicobacter pylori* Strain** | | | | | | | |
|---|---|---|---|---|---|---|---|---|
| | **LAV** **HP-8** | **LAV** **HP-9** | **LAV** **HP-10** | **LAV** **HP-11** | **LAV** **HP-12** | **LAV** **HP-13** | **LAV** **HP-14** | **LAV** **HP-15** |
| MO-EtOH | 0.195 | 0.195 | 0.195 | 0.195 | 0.39 | 0.195 | 0.195 | 0.195 |
| M0-DMSO | 0.195 | 0.39 | 0.39 | 0.195 | 0.195 | 0.09 | 0.39 | 0.09 |
| M1-EtOH | 0.39 | 0.195 | 0.195 | 0.39 | 0.195 | 0.39 | 0.195 | 0.195 |
| M1-DMSO | M | 0.78 | 0.39 | 0.195 | 0.195 | 0.195 | 0.195 | 0.39 |
| M2-EtOH | 0.39 | N/A | N/A | N/A | N/A | N/A | N/A | N/A |
| M2-DMSO | 0.39 | 0.195 | 0.195 | M | 0.39 | 0.195 | M | 0.39 |
| M3-EtOH | 0.78 | N/A | N/A | N/A | N/A | N/A | N/A | N/A |
| M3-DMSO | M | 0.39 | 0.39 | 0.39 | 0.39 | 0.39 | 0.195 | 0.78 |
| M4-EtOH | 0.06 | 0.12 | 0.12 | M | 0.12 | 0.12 | 0.24 | 0.24 |
| M4-DMSO | 0.195 | 0.39 | 0.195 | M | 0.195 | 0.195 | 0.39 | 0.195 |
| | | | | | | | | |

| **Mastic fraction** | ***Helicobacter pylori* Strain** | | | | | | | |
|---|---|---|---|---|---|---|---|---|
| | **LAV** **HP-8** | | **LAV** **HP-9** | | | | | |
| M0-EtOH | M | | N/A | | | | | |
| M0-DMSO | 0.39 | | N/A | | | | | |
| M1-EtOH | 0.39 | | N/A | | | | | |
| M1-DMSO | 0.195 | | N/A | | | | | |
| M2-EtOH | N/A | | 0.09 | | | | | |
| M2-DMSO | 0.78 | | 0.39 | | | | | |
| M3-EtOH | N/A | | 0.39 | | | | | |
| M3-DMSO | 0.39 | | M | | | | | |
| M4-EtOH | 0.12 | | 0.24 | | | | | |
| M4-DMSO | 0.195 | | 0.39 | | | | | |

As can be seen, MBCs for the various strains of *H. pylori* range from a low of 0.09 µg/mL (LAV HP-1, M0 in ethanol) to a high of 1.58 µg/mL (CCUG 3871, M3 in ethanol).

### Example 4: Determining the antimicrobial activity of mastic and its preparations

The antimicrobial activity of mastic and its preparations was tested on the following standard bacteria, which were taken from official state collections and also on clinical bacterial strains:
The strains were maintained at the Bacteriology Laboratory of the Hellenic Pasteur Institute in Brain Heart Infusion Broth (Oxoid Ltd., Basingstoke, UK), each of which contained 50% glycerol, at -70°C up to the date of use. Details of the strains are shown below in Tables 5A and 5B.

**TABLE 5A Gram (+) Strains**

| **STRAIN** | **ABBREVIATION** | **MICROBIAL ORIGIN** | **COLLECTION(*) CODE** |
|---|---|---|---|
| *Staphylococcus aureus* | Sa1 | Collection | ATCC 25923 |
| *Staphylococcus aureus* | Sa2 | Wound | H.P.I. |
| *Staphylococcus epidermidis* | Se | Wound | H.P.I. |
| *Lactobacillus casei* Shirota | Lc | Yoghurt | A.U.A. |
| *Enterococcus* spp | En | Urine | H.P.I. |

**TABLE 5B Gram (-) Strains**

| **STRAIN** | **ABBREVIATION** | **MICROBIAL ORIGIN** | **COLLECTION(*) CODE** |
|---|---|---|---|
| *Escherichia coli* | Ec1 | Collection | ATCC 25922 |
| *Escherichia coli* eae+ | Ec2 | Collection | CBIP |
| *Escherichia coli* | Ec3 | Urine | H.P.I. |
| *Pseudomonas aeruginosa* | Ps1 | Collection | ATCC 27853 |
| *Pseudomonas aeruginosa* | Ps2 | Saliva | H.P.I. |
| *Enterobacter aerogenes* | Ea | Pus | H.P.I. |
| *Salmonella* spp | Sa | Feces | H.P.I. |
| *Klebsiella* spp | KI | Urine | H.P.I. |

| | | | |
|---|---|---|---|
| ATCC = American Tissue Culture Collection CBIP = Collection de Bacteries de 1' Institut Pasteur H.P.I. = Hellenic Pasteur Institute / Bacterial collection A.U.A.= Agricultural University of Athens | | | |

### Determining the Minimum Inhibitory Concentration - MIC

Principle of the method: Every microorganism was incubated in liquid growth medium, in the presence of selected concentrations of the agent being examined. The concentration of the agent that was seen by visual observation to cause an inhibition of the microorganism growth was defined as the MIC.
Preparing the strain inocula: Each strain was re-cultured from -70°C on solid media Trypticase Soya Agar - TSA (Oxoid). After 24-hour incubation 3-5 colonies were transferred to 5 mL Mueller-Hinton Broth - MHB (Oxoid) in order to achieve a suspension with turbidity that corresponded to number 0.5 on the McFarland standard scale, as described (Clinical Microbiology Procedures handbook, 1992, Edt. H. Isenberg, American Society of Microbiology). A quantity of 0.1 mL of this suspension was transferred once more into 5 mL of MHB broth and was incubated stirred/shaken at 35°C for a period of 3-6 hours, in order for the microorganism to achieve the logarithmic growth phase. Turbidity was then adjusted using 0.85% NaCl to correspond to number 0.5 on the McFarland standard turbidity scale (approximately 1.5 x 10⁸ colony forming units - CFU/mL). A dilution of this suspension provided the standardized inoculum required for each microorganism (approximately 5 x 10⁵ CFU/mL final suspension for each well).
Preparing the agents under examination - and the microtitre trays: In order to determine the MIC sterile microtitre trays with 96 wells apiece (Sarstedt, Numbrecht, Germany) were utilized. Into each well a selected concentration of the mastic preparations being examined was added. These concentrations were prepared by dilutions of the preparations in MHB and each well received 100 µL of each dilution. The spectrum of (final) concentrations examined were 62.5, 125, 250, 500, 750, 1000, 1250 µg/mL.
Inoculation and incubation: To each well in the microtitre tray containing a dilution of the agent 10 µL of the microorganism suspension was added. Subsequently, the microtitre trays were incubated at 35°C for 24 hours.
Method Controls: In each instance the following controls took place
1. Purity Control of the initial culture
2. Growth Control of the standardized microorganism suspension by measuring the CFU/mL.
3. Sterility control
4. Control on the antimicrobial activity of the solvent (ethanol, ether, ethyl acetate, DMSO,etc, as required)
Reading the results: Where the controls were within expected limits, we proceeded to read the results. Each well in the microtitre tray was visually examined for the presence or absence of microorganism growth. The lowest concentration of the antibacterial agent that fully inhibited microorganism growth was defined as the MIC for that strain.

**TABLE 6 MIC (µg/mL) for mastic fractions used on standard and clinical Gram (+) and Gram (-) bacterial strains**

| **Mastic fraction** | | **Gram (+) bacterium** | | | | | | |
|---|---|---|---|---|---|---|---|---|
| | | **Sa1** | **Sa2** | | **Se** | **Lc** | | **En** |
| M0-EtOH | | 250 | 250 | | 250 | 250 | | 250 |
| M0-DMSO | | N/A | N/A | | N/A | N/A | | N/A |
| M1-EtOH | | 250 | 250 | | M | 250 | | 250 |
| M1-DMSO | | 250 | 250 | | 250 | 250 | | 500 |
| M2-EtOH | | 500 | 500 | | 750 | 500 | | 500 |
| M2-DMSO | | N/A | N/A | | N/A | N/A | | N/A |
| M3-EtOH | | 500 | 500 | | 1000 | 500 | | 500 |
| M3-DMSO | | 500 | 500 | | 1000 | 750 | | 500 |
| M4-EtOH | | 125 | 250 | | 250 | 250 | | 250 |
| M4-DMSO | | 250 | 250 | | M | 250 | | 250 |
| | | | | | | | | |

| **Mastic fraction** | **Gram (-) bacterium** | | | | | | | |
|---|---|---|---|---|---|---|---|---|
| | **Ec1** | **Ec2** | **Ec3** | **Ps1** | **Ps2** | **Ea** | **Ne** | **KI** |
| M0-EtOH | 250 | 250 | 250 | 250 | 500 | 250 | M | 500 |
| M0-DMSO | N/A | N/A | N/A | N/A | N/A | N/A | N/A | N/A |
| M1-EtOH | 250 | 250 | 250 | 125 | 250 | 500 | M | 500 |
| M1-DMSO | 250 | 250 | 500 | | 250 | 500 | M | 500 |
| M2-EtOH | 500 | 750 | 500 | 250 | 750 | 500 | 250 | 750 |
| M2-DMSO | ?/A | N/A | N/A | N/A | N/A | N/A | N/A | N/A |
| M3-EtOH | 500 | 500 | 500 | 750 | M | 500 | 750 | 500 |
| M3-DMSO | 750 | 500 | 500 | 750 | 500 | 500 | 750 | 750 |
| M4-EtOH | 250 | 250 | 250 | 250 | 250 | 250 | M | 250 |
| M4-DMSO | 500 | 250 | 250 | 250 | 250 | 500 | 125 | 250 |

As can be seen, MICs for the various strains of bacteria tested range from a low of 125 µg/mL (e.g., *Salmonella* 1- Sa1, M4 in ethanol) to a high of 1000 µg/mL (e.g. *Staphylococcus epidermis* - Se, M3 in ethanol).

### Determining Minimum Bactericidal Concentration - MBC

Principle of the method: Each microorganism was incubated in a liquid nutrient medium in the presence of selected concentrations of the agent being examined. Thereafter each concentration was re-cultivated on solid growth medium. The lowest concentration of the agent that caused a 99.9% lessening of the number of CFUs in comparison to the original standardized inoculum was designated as the MBC for that microorganism.

The preparation of the strain inocula, the preparation of the agents being examined and of the microtitre trays, inoculation and incubation and the necessary controls took place as in the case of ascertaining the MIC. The range of final concentrations that were examined was: 125, 250, 500, 750, 1000, 1250 and 1500 µg/mL.

In order to ascertain the MBC, the microtitre trays underwent 24-hour incubation then the content of each well was mixed with aspiration and redistribution. Subsequently 100 µL from the contents of the well were spread on the surface of a plate with agar containing blood. After 24-hour incubation at 35°C, the results were read.
Reading the results: Each re-cultivation plate was examined and the number of colonies produced was noted. Plates were mentioned if they contained up to 300 CFU. The lowest concentration of the agent that caused a reduction in the number of CFUs greater or equal to 99.9% in comparison to the original standardized inoculum was designated as the MBC for that inoculum.

**TABLE 7 MBC (µg/mL) for mastic fractions used on standard and clinical Gram (+) and Gram (-) bacterial strains**

| **Mastic fraction** | **Gram (+) bacterium** | | | | | | | |
|---|---|---|---|---|---|---|---|---|
| | **Sa1** | | **Sa2** | **Se** | | **Lc** | **En** | |
| M0-EtOH | 750 | | 750 | 1000 | | 750 | 1000 | |
| M0-DMSO | N/A | | N/A | N/A | | N/A | N/A | |
| M1-EtOH | 750 | | 1000 | 1000 | | 750 | 1000 | |
| M1-DMSO | 750 | | 1000 | 1000 | | 1000 | 1000 | |
| M2-EtOH | 1000 | | 10500 | 1500 | | 1000 | 1500 | |
| M2-DMSO | N/A | | N/A | N/A | | N/A | N/A | |
| M3-EtOH | 1500 | | 1500 | M | | 1000 | 2000 | |
| M3-DMSO | 1500 | | 2000 | 1500 | | 1000 | 2000 | |
| M4-EtOH | 750 | | 750 | M | | 750 | 1000 | |
| M4-DMSO | 1000 | | 750 | 750 | | 750 | 1000 | |
| | | | | | | | | |

| **Mastic fraction** | **Gram (-) bacterium** | | | | | | | |
|---|---|---|---|---|---|---|---|---|
| | **Ec1** | **Ec2** | **Ec3** | **Ps1** | **Ps2** | **Ea** | **Ne** | **K1** |
| M0-EtOH | 1000 | 750 | 1000 | 1000 | 1000 | 750 | M | 750 |
| M0-DMSO | N/A | N/A | N/A | N/A | N/A | N/A | N/A | N/A |
| M1-EtOH | 1000 | 1000 | 15000 | 750 | 1000 | 1000 | M | 1000 |
| M1-DMSO | 1500 | 1000 | 1500 | M | 1500 | 1000 | M | 1000 |
| M2-EtOH | 1500 | 1500 | 1500 | 1000 | 1500 | | 1000 | 1000 |
| M2-DMSO | N/A | N/A | N/A | N/A | N/A | N/A | N/A | N/A |
| M3-EtOH | 1500 | 1500 | 2000 | 750 | 2000 | 1500 | 1000 | 1500 |
| M3-DMSO | 1500 | 1500 | 2000 | 1000 | 2000 | M | 15000 | 2000 |
| M4-EtOH | 1000 | 750 | 750 | M | 1000 | 750 | 1000 | 750 |
| M4-DMSO | 1000 | 1000 | 750 | M | 1000 | 1000 | 1000 | 1000 |

As can be seen, MBCs for the various strains of bacteria tested range from a low of 750 µg/mL (e.g., *Salmonella* 1 - Sa1, M0 in ethanol) to a high of 2000 µg/mL (e.g. *Salmonella* 2 - Sa2, M3 in DMSO).

### Example 5: Determining the antimicrobial activity of mastic on Propionibacterium acnes

In order to examine the antimicrobial activity we determined the Minimum Inhibitory Concentration - MIC - and the Minimum Bactericidal Concentration - MBC.

### Determining the Minimum Inhibitory Concentration - MIC

Preparing the strain inoculum: The strain of *Propionibacterium acnes* CIP 53117 was recultured from - 70°C where it was kept on a plate with blood-containing anaerobic culture medium [Brucella Broth (BBL) with Bacto Agar 0.5% (Difco Laboratories, Detroit, Michigan, USA) with Vitamin K, 1 µg/mL (Sigma-Aldrich Ltd, St. Louis, Missouri, USA, haemine 5 µg/mL (Sigma-Aldrich Ltd) and 5% horse blood]. The incubation took place inside anaerobic condition flasks (BBL GasPakPlus, Becton Dickinson, Sparks, MD, USA) for 48 hours. After the incubation, 3-5 colonies were transferred to 5 mL of pre-reduced thioglycolic broth (Oxoid). This was incubated until the microorganism in the broth reached a level of turbidity comparable to number 0.5 of the McFarland scale (approximately 1.5 x 10⁸ CFU/mL). This bacterial suspension was diluted 1/15 in order to achieve a final standardised bacterial suspension (1 x 10⁶ CFU/mL) in each microtitre tray well (see below).
Preparing the agents under examination - and the microtitre trays: In order to determine the MIC sterile microtitre trays with 96 wells apiece (Sarstedt, Numbrecht, Germany) were utilized. A selected concentration of the mastic preparations being examined dissolved in 100 µL of thioglycolic broth was added into each well. The range of (final) concentrations examined was 0.06, 0.12, 0.24, 0.48, 0.96 and 1.92 mg/mL. To each well in the microtitre tray containing a dilution of agent was done by addition of 10 µL of the standardised bacterial suspension. Subsequently, the microtitre trays were incubated at 35°C for 48 hours in anaerobic conditions, as above.
Method Controls: In each instance the following controls took place
1. Purity Control of the initial culture (culturing in both aerobic and anaerobic conditions)
2. Growth Control of the standardized microorganism suspension by measuring the CFU/mL.
3. Sterility control
4. Control on the antimicrobial activity of the solvent (ethanol, ether, ethyl acetate, DMSO,etc, as required)
Reading the results: Where the controls were within expected limits, we proceeded to read the results. Each well in the microtitre tray was visually examined for the growth or not of the microorganism. The lowest concentration of the antibacterial agent that fully inhibited microorganism growth was defined as the MIC for that strain.

### Determining the Minimum Bactericidal Concentration - MBC

The preparation of the *Propionibacterium acnes* strain inocula, the preparation of the agents being examined and of the microtitre trays, the inoculation and the incubation and the necessary controls took place as in the case of ascertaining the MIC. The range of final concentrations that were examined was: 0.06, 0.12, 0.24, 0.48, 0.96, 1.92 and 3.84 mg/mL. In order to ascertain the MBC, the microtitre trays underwent 24-hour incubation then the content of each well was mixed by aspiration and redistribution. Subsequently 100 µL from the contents of the well were spread on the surface of a plate with blood-containing anaerobic culture medium (see above). There followed 48-hour incubation at 35°C in microaerobic conditions after which the results were read. Reading the results: Each re-cultivation plate was examined and the number of colonies produced was noted. Plates were mentioned if they contained up to 300 CFU. The lowest concentration of the agent that caused a reduction in the number of CFUs greater or equal to 99.9% in comparison to the original standardized inoculum was designated as the MBC for that inoculum.

**TABLE 8 Determining the antimicrobial activity of mastic and its fractions against Propionibacterium acnes CIP 53117**

| **Mastic fraction** | **Strain *Propionibacterium acnes CIP 53117*** | |
|---|---|---|
| | **MIC (**µ**g/mL)** | **MBC (**µ**g/mL)** |
| M0-EtOH | 240 | 480 |
| M0-DMSO | 240 | 480 |
| M1-EtOH | 240 | 480 |
| M1-DMSO | 240 | 960 |
| M2-EtOH | 480 | 960 |
| M2-DMSO | N/A | N/A |
| M3-EtOH | 480 | 960 |
| M3-DMSO | 960 | 960 |
| M4-EtOH | 120 | 240 |
| M4-DMSO | 240 | 240 |

As can be seen, MBCs for the Propionibacterium strain tested range from a low of 120 µg/mL (M4 in ethanol) to a high of 960 µg/mL (M3 in DMSO).

### Example 6: Cytotoxic activity of extracts of Pistacia lentiscus

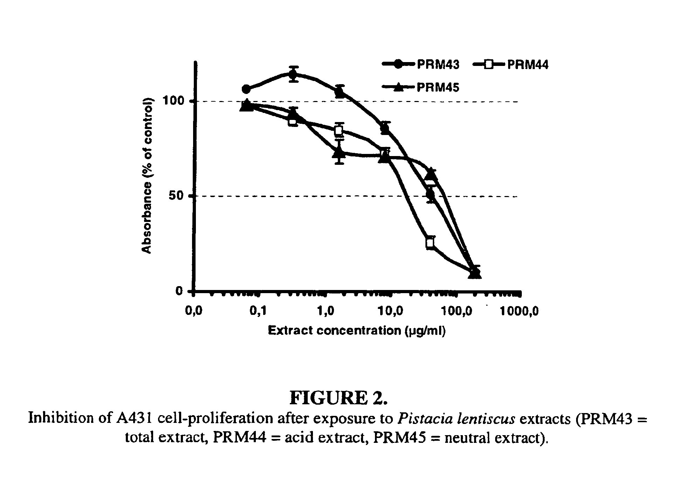

Three extracts derived from *Pistacia lentiscus* var. *Chia* were tested for their cytotoxic/cytostatic activity against three established cancer cell lines, i.e human mammary adenocarcinoma MCF-7, human colon adenocarcinoma HT-29, and human epidermoid carcinoma A431. A modification of the MTT method (Denizot and Lang, 1986, J Immunol Meth 89, 271) was used for the assessment of cytotoxicity. Briefly, exponentially growing cells were exposed to varying concentrations of the test compounds (or control medium) for 48 hours. Then, the medium was replaced with MTT [3-(4,5-dimethylthiazol-2-yl)-2,5-diphenyltetrazolium bromide] for a further 4 -hour incubation. Finally, the MTT-formazan was solubilized in isopropanol and the optical density was measured at a wavelength of 550 nm and a reference wavelength of 690 nm. A representative example of the semi-log inhibition curves obtained after exposure of A431 cells to the three *Pistacia lentiscus* extracts is illustrated in Fig. 2.

The inhibition curves were used to determine the IC₅₀ of each extract, i.e. the concentration that produced a 50% reduction of the absorbance relative to the absorbance of the control culture. The average IC₅₀s of each extract for each cell line, after at least three independent trials, are presented in the following table (5-fluorouracil a classical chemotherapeutic agent, was included as a positive control in the case of A431 epidermoid carcinoma cells):

**TABLE 9. Inhibition of cancer cell proliferation after exposure to Pistacia lentiscus extracts (IC₅₀ values in µg/mL)**

| **Cell lines:** | **Epidermoid carcinoma** **A431** | **Colon adenocarcinoma** **HT-29** | **Mammary adenocarcinoma** **MCF-7** |
|---|---|---|---|
| **Extracts:** | | | |
| **PRM43 (total)** | **30 (± 14)** | **62 (± 22)** | **44 (± 5)** |
| **PRM44 (acid)** | **17 (± 2)** | **48 (± 11)** | **25 (± 5)** |
| **PRM45 (neutral)** | **60 (± 0.1)** | **100 (± 10)** | **88 (± 28)** |
| **5-fluorouracil** | **2.25 (± 0.35)** | | |

| | | | |
|---|---|---|---|
| The numbers in parentheses indicate standard deviations | | | |

The above results indicate that all three extracts possess a considerable non-selective inhibitory activity against the three cancer cell lines, with IC₅₀s in the range of 10 to100 µg/mL. In all tested cell lines, the agent with the highest activity was the acid extract (PRM44). The cell line that proved most active as shown above with respect to all three agents was A431, derived from an epidermoid carcinoma

### Example 7: Cytotoxic activity of isomasticadienonic acid, isomasticadienolic acid, oleanonic acid and moronic acid extracts from Pistacia lentiscus

We used the methodology of MTT (see Example 6), using A431 cells. The average IC₅₀s of each compound, after at least three independent trials, are presented in the following table (the data of the previous report regarding A431 cells are included in the table for reasons of comparison):

**TABLE 10. Inhibition of A431 cancer cell proliferation by Pistacia lentiscus extracts (IC₅₀ values in µg/mL) and isolated constituents (IC₅₀ values in µg/mL & µM)**

| **Extracts:** | **µg/mL** | **Constituents:** | **µg/mL** | **µM** |
|---|---|---|---|---|
| **PRM43** **(total)** | **30 (± 14)** | **Isomasticadienonic** | **51.3 (± 18.7)** | **113 (± 41.1)** |
| **PRM44** **(acid)** | **17 (± 2)** | **Isomasticadienolic** | **24 (± 9.3)** | **53.3 (± 20.5)** |
| **PRM45** **(neutral)** | **60 (± 0.1)** | **oleanonic** | **17.5 (± 2.8)** | **38.3 (± 6.2)** |
| **Essential Oil** | **2.45 (± 0.05)** | **moronic** | **47.7 (± 6.8)** | **105 (± 15)** |
| | **5-fluorouracil** | | **2.25 (± 0.35)** | **17.3 (± 2.7)** |

| | | | | |
|---|---|---|---|---|
| The numbers in parentheses indicate standard deviations | | | | |

The above results show that:
1) The essential oil of *Pistacia lentiscus* possessed a remarkable inhibitory activity with IC₅₀ approximately one order of magnitude lower than the other three extracts.
2) The four isolated ingredients of *Pistacia lentiscus* exhibited varying degrees of activity Oleanonic acid, with an IC₅₀ approximately two-fold of that of 5-fluorouracil in terms of µM (micromoles), was regarded as the most promising.

### Example 8: Cytotoxic activity of Pistacia lentiscus extracts

The neutral extract of the essential oil and two of the isolated ingredients, i.e. isomasticadienolic acid, and oleanonic acid were tested for cytoprotective activity using the methodology of MTT (Example 7) and three established human cancer cell lines. The average IC₅₀s of each compound, after two independent trials, are presented in the following table:

**TABLE 11. Inhibition of cancer cell proliferation after exposure to Pistacia lentiscus extracts and isolated constituents**

| | **Cell lines** | | |
|---|---|---|---|
| | **Epidermoid carcinoma** **A431** | **Colon adenocarcinoma** **HT-29** | **Lung Carcinoma** **A549** |
| **Neutral extract** | **31 (±9)** | **92.5 (± 12.5)** | **33.5 (± 6.5)** |
| **Essential oil** | **0.3 (± 0.02)** | **0.6 (± 0.2)** | **0.5 (± 0.1)** |
| **Isomasticadienolic** | **25 (±5)** | **95 (± 5)** | **95 (± 5)** |
| **Oleanonic** | **45 (±5)** | **105 (± 5)** | **110 (± 0.0)** |

| | | | |
|---|---|---|---|
| The numbers indicate the average IC₅₀ values (µg/mL) (± standard deviation) | | | |

In general, the above results were in agreement with those of Examples 6 and 7. The lower value (compared to the previous report) determined for the neutral extract in A431 cells, as well as, the higher values for oleanonic acid in A431 and for the neutral extract in HT-29 were within the limits of the experimental variations observed in *in vitro* cytotoxicity experiments. However, the essential oil repeatedly demonstrated remarkable inhibitory activity and was even more intense in these trials. One explanation for the shift of one order of magnitude [compare the value of 2.45 (± 0.05) for A431 cells of the previous example to the present 0.3 (± 0.02)] in the present set of trials is that we used absolute ethanol to dissolve the essential oil in this experiment. We selected ethanol as a solvent once the properties of the constituents of the essential oil (i.e. monoterpenes such as α-pinene, β-myrcene etc.) were known, which exhibit good solubility in ethanol. The above experimental data (Table 11) illustrate the use of essential oil extracts of *Pistacia lentiscus* as a cytotoxic/cytostatic agent where these essential oil extracts have IC₅₀ values of around 500 ng/mL against all three cancer cell lines treated with essential oil.

### Example 9: Preparation of a delivery system for administering active agent

In (a) to (h), formulations are provided for administering active agent as described.

### (a) Formulations for orally delivering mastic whole extract

**TABLE 12 Compositions of example tablet formulations containing whole extract and optionally essential oil**

| **Material** | **Weight (mg)** |
|---|---|
| Mastic gum whole extract | 20-250 |
| Mastic gum essential oil | 0-5 |
| Excipients ¹ | qsp ² |

| | |
|---|---|
| ¹ Fillers, lubricants, glidants, colourants, anti-oxidants, preservatives, etc ² To fill a 400mg capsule or to be compressed to a tablet | |

### (b) Formulations for orally delivering mastic acid extract

**TABLE 13 Compositions of example tablet formulations containing acid extract and optionally essential oil**

| **Material** | **Weight (mg)** |
|---|---|
| Mastic gum acid extract | 5-100 |
| Mastic gum essential oil | 0-5 |
| Excipients¹ | qsp ² |

| | |
|---|---|
| ¹ Fillers, lubricants, glidants, colorants, anti-oxidants, preservatives, etc ² To fill a 400mg capsule or to be compressed to a tablet | |

### (c) Formulations for orally delivering mastic neutral extract

**TABLE 14 Compositions of example tablet formulations containing neutral extract and optionally essential oil**

| **Material** | **Weight (mg)** |
|---|---|
| Mastic gum neutral extract | 20-250 |
| Mastic gum essential oil | 0-5 |
| Excipients ¹ | qsp ² |

| | |
|---|---|
| ¹ Fillers, lubricants, glidants, colourants, anti-oxidants, preservatives, etc ² To fill a 400mg capsule or to be compressed to a tablet | |

### (d) Formulations for orally delivering mastic isolated components

**TABLE 15 Compositions of example tablet formulations containing mastic isolated components and optionally essential oil**

| **Material** | **Weight (mg)** |
|---|---|
| Mastic gum isolated component ³ | 5-80 |
| Mastic gum essential oil | 0-5 |
| Excipients ¹ | qsp ² |

| | |
|---|---|
| ¹ Fillers, lubricants, glidants, colorants, anti-oxidants, preservatives, etc ² To fill a 400mg capsule or to be compressed to a tablet ³ Masticadienonic acid, aldehyde or alcohol; masticadienolic acid, aldehyde or alcohol; isomasticadienonic acid aldehyde or alcohol; isomasticadienolic acid, aldehyde or alcohol; oleanonic acid, aldehyde or alcohol; moronic acid, aldehyde or alcohol; α-pinene; β-myrcene; tirucallol; betulonal. | |

### (e) Cream formulations for topically delivering mastic whole extract

**TABLE 16 Percent compositions of example cream formulations containing mastic whole extract, acid extract, or neutral extract and optionally essential oil**

| **Material** | **%** |
|---|---|
| Mastic whole, acid or neutral extract | 2-20 |
| Mastic gum essential oil | 0-5 |
| Cream base | qsp |

### (f) Cream formulations for topically delivering mastic isolated components

**TABLE 17 Percent compositions of example cream formulations containing mastic isolated components and optionally essential oil**

| **Material** | **%** |
|---|---|
| Mastic isolated component ¹ | 1-10 |
| Mastic gum essential oil | 0-5 |
| Cream base | qsp |

| | |
|---|---|
| ¹ Masticadienonic acid, aldehyde or alcohol; masticadienolic acid, aldehyde or alcohol; isomasticadienonic acid aldehyde or alcohol; isomasticadienolic acid, aldehyde or alcohol; oleanonic acid, aldehyde or alcohol; moronic acid, aldehyde or alcohol; α-pinene; β-myrcene; tirucallol; betulonal. | |

### (g) Shampoo formulations for topically delivering mastic whole extract

**TABLE 18 Percent compositions of example shampoo formulations containing mastic whole extract and optionally essential oil**

| **Material** | **%** |
|---|---|
| Mastic whole extract | 1-25 |
| Mastic gum essential oil | 0-5 |
| Shampoo base | qsp |

### (h) Shampoo formulations for topically delivering mastic isolated components

**TABLE 19 Percent compositions of example shampoo formulations containing mastic isolated components and optionally essential oil**

| **Material** | **%** |
|---|---|
| Mastic isolated component¹ | 1-10 |
| Mastic gum essential oil | 0-5 |
| Shampoo base | qsp |

| | |
|---|---|
| ¹ Masticadienonic acid, aldehyde or alcohol; masticadienolic acid, aldehyde or alcohol; isomasticadienonic acid aldehyde or alcohol; isomasticadienolic acid, aldehyde or alcohol; oleanonic acid, aldehyde or alcohol; moronic acid, aldehyde or alcohol; α-pinene; β-myrcene; tirucallol; betulonal. | |

Formulations of mastic extract and isolated components are used to treat microbial infections *in vivo,* particularly *H. pylori* infections, *Staphylococcus aureus* and *Staphylococcus epidermis, Pseudomonas aeruginosa*, and *Propionibacterium acnes* infections, according to examples 11-14, below. Formulations of mastic extract and isolated components are used to treat cell hyperproliferation such as dandruff, according to Examples 15 and 16.

### Example 10: Mastic components For Treatment of H. Pylori Gastric Colonization

In order to demonstrate the antimicrobial property (capacity) of mastic gum against *H. pylori in vivo*, whole mastic extract was continuously delivered in potable water at a dose of 0.75mg per day, to mice that had been infected with *H. pylori.* Over a period of the passage of three (3) months, the degree of colonization of *H. pylori* was histopathologically evaluated in the andrum and the stomach body ("corpus") according to Lausanne (Lee et al 1997) criteria. Also, the degree and the activity of gastritis (criteria under Sydney, Dixon et al 1996) were evaluated. Similar experiments can be done with acid fraction extract and neutral fraction extract.

### RESULTS

The results of the microbiological control of the gastric samples and PCR are presented below in Table 20.

**TABLE 20**

| Groups | Animal code - | Rapid Urease Test per culture | Isolation PCR | 1st | 2nd | 3rd | 4th | 5th |
|---|---|---|---|---|---|---|---|---|
| Hp(+) | SH1 | + | + | + | NA | NA | + | + |
| Mx(-) | SH2 | + | + | - | NA | NA | - | + |
| | SH3 | + | + | + | NA | NA | + | + |
| | SH4 | + | + | + | - | NA | - | + |
| | SH5 | + | + | + | NA | NA | + | + |
| | SH6 | + | + | - | - | NA | - | + |
| | SH7 | - | - | - | NA | NA | - | - |
| | SH8 | + | + | + | - | - | - | + |
| | SH9 | + | + | NA | NA | NA | + | + |
| | SH10 | + | + | - | - | | - | + |
| Hp(-) | SM1 | - | NA | NA | NA | NA | - | - |
| Mx(+) | SM2 | - | NA | NA | NA | NA | - | - |
| | SM3 | - | NA | NA | NA | NA | - | - |
| | SM4 | - | NA | NA | NA | NA | - | - |
| | SM5 | - | NA | NA | NA | NA | - | - |
| | SM6 | - | NA | NA | NA | NA | - | - |
| | SM7 | - | NA | NA | NA | NA | - | - |
| | SM8 | - | NA | NA | NA | NA | - | - |
| | SM9 | - | NA | NA | NA | NA | - | - |
| | SM10 | - | NA | NA | NA | NA | - | - |

| Groups | Animal code - | Rapid Urease Test per culture | Isolation PCR | 1st | 2nd | 3rd | 4th | 5th |
|---|---|---|---|---|---|---|---|---|
| Hp(+) | SMH1 | + | + | + | - | - | + | + |
| Mx(+) | SMH2 | + | + | - | - | - | - | + |
| | SMH3 | + | - | - | NA | NA | - | + |
| | SMH4 | + | - | - | NA | NA | - | + |
| | SMH5 | + | + | + | + | + | + | + |
| | SMH6 | - | + | - | NA | NA | - | + |
| | SMH7 | + | - | - | NA | NA | - | + |
| | SMH8 | - | - | - | - | - | - | - |
| | SMH9 | + | - | + | - | - | - | + |
| | SMH10 | - | - | + | - | - | - | + |

The presence of *H. pylori* in gastric samples was detected with PCR in 18 of 20 cases, where possible, by strain isolation through culture after repeated re-cultures of gastric samples in 7 of 20 cases and with simultaneous urease test on partial cultures (18/20). The PCR method, considered the most sensitive method, detected the presence of *H. pylori* in most of the cases. *H. pylori* was not detectable in the negative group (where the mice were not infected with *H. pylori*).

### HISTOLOGICAL EVALUATION

In Table 21 are presented the data from the histological evaluation of gastric samples in terms of *H. pylori* colonization, degree of gastritis and the activity in the tissue.

**Table 21**

| | Gastric antrum | | | Gastric corpus | | |
|---|---|---|---|---|---|---|
| Animal code | Gastritis Degree | Activity | *H. pylori* Colonies forming | Gastritis Degree | Activity | *H. pylori* Colonies forming |
| SH1 | 1 | 1 | 2 | 0 | 0 | 1 |
| SH2 | 1 | 0 | 3 | 1 | 0 | 1 |
| SH3 | 1 | 1 | 3 | 1 | 0 | 1 |
| SH4 | 2 | 2 | 1 | 1 | 0 | 0 |
| SH5 | ΔA | ΔA | ΔA | 1 | 0 | 2 |
| SH6 | 1 | 1 | 1 | 1 | 0 | 0 |
| SH7 | 1 | 2 | 0 | 0 | 0 | 0 |
| SH8 | 1 | 1 | 2 | 0 | 0 | 0 |
| SH9 | 1 | 1 | 1 | 1 | 1 | 0 |
| SH10 | 1 | 1 | 2 | 1 | 1 | 1 |
| SM1 | 0 | 0 | 0 | 0 | 0 | 0 |
| SM2 | 0 | 0 | 0 | 0 | 0 | 0 |
| SM3 | 0 | 0 | 0 | 0 | 0 | 0 |
| SM4 | 0 | 0 | 0 | 0 | 0 | 0 |
| SM5 | 0 | 0 | 0 | 0 | 0 | 0 |
| SM6 | 0 | 0 | 0 | 0 | 0 | 0 |
| SM7 | 0 | 0 | 0 | 0 | 0 | 0 |
| SM8 | 0 | 0 | 0 | 0 | 0 | 0 |
| SM9 | 0 | 0 | 0 | 0 | 0 | 0 |
| SM10 | 0 | 0 | 0 | 0 | 0 | 0 |
| SMH1 | 1 | 0 | 2 | 0 | 0 | 0 |
| SMH2 | 1 | 1 | 1 | 0 | 0 | 0 |
| SMH3 | 1 | 1 | 1 | 0 | 0 | 0 |
| SMH4 | 2 | 2 | 0 | 0 | 0 | 0 |
| SMH5 | 1 | 2 | 1 | 1 | 2 | 0 |
| SMH6 | 1 | 0 | 0 | 0 | 0 | 0 |
| SMH7 | 2 | 2 | 1 | 1 | 1 | 0 |
| SMH8 | 1 | 0 | 0 | 1 | 0 | 0 |
| SMH9 | 1 | 0 | 1 | 0 | 0 | 0 |
| SMH10 | 1 | 0 | 1 | 0 | 0 | 0 |

| | | | | | | |
|---|---|---|---|---|---|---|
| ΔA: Not appropriate sample | | | | | | |

### Observations:

In the mice from the negative control group that received only whole mastic extract (i.e. not infected with *H. pylori*), as expected, neither *H. pylori* nor gastritis was detectable. No deteriorations from the continuous delivery of control formulations were observed in the analyzed samples from any control mouse. By contrast, in the mice from the positive control group (i.e., those mice positive for *H. pylori* infection), as expected *H. pylori* was identified on the surface of the mucosa and the epithelium before treatment. In a blow-up, the bacteria appeared to be attached to the gastric epithilium. The colonies-forming density was between medium and high (grade 2-3) in the antrum, but very limited in the corpus (grade 1).

The secondary condition of chronic active gastritis, similar to the human form, was induced and developed in mice by infection with *H. pylori.* The grade (chronic presence of cellular populations of the inflammation) and the activity (tissue filtration from neutralists) took the grades 1 and 2 based on the Sydney's range. These results were in accordance with the image described for the *in vivo* model of the experimental infection *H. pylori* SS 1 (Lee et al 1997).

In the study group SMH (*H. pylori*-infected mice treated with whole mastic extract) it was concluded that, from the onset of the experiment to the conclusion three-months later, a relative decrease of the number of the observed bacteria in the epithelium and epithelial depressions occurred.

A statistic analysis was done using the method Wilcoxon Rank Sum Test (Ilstrup 1990) and the results are presented below in the Table 22 for the "antrum" and Table 23 for "corpus", respectively.

**TABLE 22 "Antrum"**

| Hp Colonies forming | | | | | |
|---|---|---|---|---|---|
| | Grade0 | Grade1 | Grade2 | Grade3 | Total |
| SH Group | 1 | 3 | 3 | 2 | 9 |
| SMH Group | 3 | 6 | 1 | 0 | 10 |
| Total | 4 | 9 | 4 | 2 | 19 |
| Average Rank | 2.5 | 9 | 15.5 | 18.5 | |
| Rank sum SH | 113 | | | | |
| Rank sum SMH | 77 | | | | |
| Z 1.92 p=0.0274 p<0.05 Statistically important difference at level 95% | | | | | |
| | | | | | |

| Gastritis grade | | | | | |
|---|---|---|---|---|---|
| | Grade0 | Grade1 | Grade2 | | Total |
| SH Group | 0 | 8 | 1 | | 9 |
| SMH Group | 0 | 8 | 2 | | 10 |
| Total | 0 | 16 | 3 | | 19 |
| Average Rank | 0 | 8.5 | 18 | | |
| Rank sum SH 86 | | | | | |
| Rank sum SMH | 104 | | | | |
| Z 0.37 No statistically significant difference | | | | | |
| | | | | | |

| Gastritis activity | | | | | |
|---|---|---|---|---|---|
| | Grade0 | Grade1 | Grade2 | | Total |
| SH Group | 1 | 6 | 2 | | 9 |
| SMH Group | 5 | 2 | 3 | | 10 |
| Total | 6 | 8 | 5 | | 19 |
| Average Rank | 3.5 | 10.5 | 17 | | |
| Rank sum SH | 100.5 | | | | |
| Rank sum SMH | 89.5 | | | | |
| Z 0.90 No statistically significant difference | | | | | |

**TABLE 23 "Corpus"**

| Hp Colonies forming | | | | | |
|---|---|---|---|---|---|
| | Grade0 | Grade1 | Grade2 | Grade3 | Total |
| SH Group | 5 | 4 | 1 | 0 | 10 |
| SMH Group | 10 | 0 | 0 | 0 | 10 |
| Total | 15 | 4 | 1 | 0 | 20 |
| Average | 8 | 17.5 | 20 | | |
| Rank | | | | | |
| Rank sum SH | 130 | | | | |
| Rank sum SMH | 80 | | | | |
| Z 1.93 p=0.0268 p<0.05 Statistically significant difference at level 95% | | | | | |
| | | | | | |

| Gastritis grade | | | | | |
|---|---|---|---|---|---|
| | Grade0 | Grade1 | Grade2 | | Total |
| SH Group | 3 | 7 | 0 | | 10 |
| SMH Group | 7 | 3 | 0 | | 10 |
| Total | 10 | 10 | 0 | | 20 |
| Average Rank | 5.5 | 15.5 | | | |
| Rank sum SH | 125 | | | | |
| Rank sum SMH | 85 | | | | |
| Z 1.54 Statistically not important difference | | | | | |
| | | | | | |

| Gastritis activity | | | | | |
|---|---|---|---|---|---|
| | Grade0 | Grade1 | Grade2 | | Total |
| SH Group | 8 | 2 | 0 | | 10 |
| SMH Group | 8 | 1 | 1 | | 10 |
| Total | 16 | 3 | 1 | | 20 |
| Average Rank | 8.5 | 18 | 20 | | |
| Rank sum SH | 104 | | | | |
| Rank sum SMH | 106 | | | | |
| Z 0.11 Statistically not important difference | | | | | |

After the statistical processing, a clear decrease of the Colonies-forming grade of *H. pylori* both in "antrum" and in stomach "corpus" was revealed. On the contrary, for the mice taken as a whole, there was not observed any disappearance of the phenomenon of gastritis, neither in the antrum, nor in the corpus. It is possible that a reduction in the secondary condition of gastritis requires extended treatment and/or higher dosages, which can be optimized by experimentation utilizing more extensive variations in dosage and treatment type. The present *in vivo* results augmented the antimicrobial activity observed *in vitro* against *H. pylori* and suggest that extrapolation to *in vivo* treatment of other animals, including humans, with other microbial infections is supported experimentally.

In addition, it is envisioned that further optimization of dosage and treatment times including higher doses and longer treatments, will further reduce *H. pylori* bacterial count *in vivo,* with concomitant reduction in the secondary condition of gastritis.

Further, as with the experiments which verified the efficacy of using mastic extracts to reduce *H. pylori* infections *in vivo* based on the suggestion from the results of experiments performed *in vitro* (compare the results of Example 10 - *in vivo* - with the results of Examples 6-8 *- in vitro* results using four human cancer cell lines) it is analogously predicted that the efficacy of using mastic extracts, essential oil and/or components to reduce other microbial infections will carry through for *in vivo* treatments. Protocols for such *in vivo* treatments are described below in Examples 11-16.

### Example 11: Use of Mastic Extracts or Essential Oil For In Vivo (Human) Treatment of H. Pylori Infections

Dilutions of whole extract product, acid fraction product, neutral fraction product, or essential oil preparations are delivered orally, for example in tablet form, from once to four times daily in total doses ranging from about 1 g to 5 g per day to human test subjects (groups of 10 to 20 subjects per study for both control groups and those taking mastic compounds) infected with a variety of *H. pylori* strains (see Example 3 and Table 3, above).

After total treatment times varying from about one-half month to up to six months or longer, as needed, the degree of colonization of bacteria is histopathologically evaluated by taking samples of the infected tissue and surrounding area, following established protocols. Also, the grade (chronic present of cellular populations of inflammation) and the activity (tissue filtration from neutralists) of inflammatory conditions associated with *H. pylori* infections, such as gastritis is evaluated and compared to humans treated in similar kind with a placebo, and to the levels of such conditions in the test subjects pre-treatment.

For control groups, tablets containing all ingredients in the mastic extract or essential oil formulations except the mastic components (for example, required solvents, stabilizers, glidants, emulsifiers, colorants, preservatives, etc.) are given to subjects under identical conditions at identical times each day for identical total study time (i.e. ½-month, one-month, two-months, three-months, etc. up to six-months).

Similarly, control groups receiving a placebo (for example, a glucose tablet) are also evaluated for degree of colonization and activity of related inflammatory conditions, and compared to the treatment groups.

### Example 12: Use of Mastic Isolated Components For In Vivo (Human) Treatment of H. Pylori Infections

Dilutions of mastic isolated components, for example masticadienonic acid, masticadienolic acid, isomasticadienonic acid, isomasticadienolic acid, oleanonic acid, and moronic acid, are delivered orally, for example in tablet form, from once to four times daily in total doses ranging from about 1 g to 5 g per day to human test subjects (groups of 10 to 20 subjects per study for both control groups and those taking mastic compounds) infected with a variety of *H. pylori* strains (see Example 3 and Table 3, above).

After total treatment times varying from about one-half month to up to six months or longer, as needed, the degree of colonization of bacteria is histopathologically evaluated by taking samples of the infected tissue and surrounding area, following established protocols. Also, the grade (chronic present of cellular populations of inflammation) and the activity (tissue filtration from neutralists) of inflammatory conditions associated with *H. pylori* infections, such as gastritis is evaluated and compared to humans treated in similar kind with a placebo, and to the levels of such conditions in the test subjects pre-treatment.

For control groups, tablets containing all ingredients in the mastic isolated components formulations, except the mastic isolated components (for example, required solvents, stabilizers, glidants, emulsifiers, colorants, preservatives, etc.) are given to subjects under identical conditions at identical times each day for identical total study time (i.e. ½-month, one-month, two-months, three-months, etc. up to six-months).

Similarly, control groups receiving a placebo (for example, a glucose tablet) are also evaluated for degree of colonization and activity of related inflammatory conditions, and compared to the treatment groups.

### Example 13: Use of Mastic Extract or Essential Oil For In Vivo (Human) Treatment of Propionibacterium, Staphylococcus, and Pseudomonas Infections

Dilutions of whole extract product, acid fraction product, neutral fraction product and optionally essential oil preparations are topically applied from once to five times daily in a cream, ointment, solution, lotion, tincture, shampoo, patch, film, hydrogel or gel containing about 2 % up to about 20 % active component, and optionally from about 1 to 5% essential oil, to human test subjects (groups of 10 to 20 subjects per study for both control groups and those applying mastic compounds) infected with any of a variety of *Propionibacterium acnes* strains, including *Propionibacterium acnes* CBIP 53117 (see Example 2B, above), or to human test subjects infected with any of a variety of *Staphylococcus aureus, Staphylococcus epidermis*, and *Pseudomonas aeruginosa* strains.

Treatment times may vary from a range of a total of about one-half month or up to six months or longer, as needed to treat the infection. Following treatment, the degree of colonization of bacteria is histopathologically evaluated by taking samples of the infected tissue and surrounding area, following established protocols. Also, the degree and the activity of secondary conditions such as dermatitis, lesions and the like associated with *Propionibacterium, Staphylococcus* and *Pseudomonas* infections are evaluated and compared to humans treated in similar kind with a placebo, and compared to the levels of such conditions in the test subjects pre-treatment.

For control groups, creams (or other topical formulations) containing all ingredients of the mastic extract or essential oil formulations, except the mastic components (for example, fatty acids, oils, stabilizers, emulsifiers, pigments, fragrances, etc.) are applied by subjects under identical conditions at identical times each day for identical total study time (lie. ½-month, one-month, two-months, three-months, etc. up to six-months).

Similarly, control groups receiving just the cream or other topical formulation components, are also evaluated for degree of colonization and activity of related secondary conditions, and compared to the treatment groups.

### Example 14: Use of Mastic Isolated Components For In Vivo (Human) Treatment of Propionibacterium, Staphylococcus, and Pseudomonas Infections

Dilutions of mastic isolated components, for example masticadienonic acid, masticadienolic acid, isomasticadienonic acid, isomasticadienolic acid, oleanonic acid, and moronic acid, are topically applied from once to five times daily in a cream, ointment, solution, lotion, tincture, shampoo, patch, film, hydrogel or gel containing about 1 % up to about 10 % active component, and optionally from about 1 to 5% essential oil, to human test subjects (groups of 10 to 20 subjects per study for both control groups and those applying mastic compounds) infected with any of a variety of *Propionibacterium acnes* strains, including *Propionibacterium acnes* CBIP 53117 (see Example 2B, above), or to human test subjects infected with any of a variety of *Staphylococcus aureus, Staphylococcus epidermis*, and *Pseudomonas aeruginosa* strains.

Treatment times may vary from a range of a total of about one-half month or up to six months or longer, as needed to treat the infection. Following treatment, the degree of colonization of bacteria is histopathologically evaluated by taking samples of the infected tissue and surrounding area, following established protocols. Also, the degree and the activity of secondary conditions such as dermatitis, lesions and the like associated with *Propionibacterium, Staphylococcus* and *Pseudomonas* infections are evaluated and compared to humans treated in similar kind with a placebo, and compared to the levels of such conditions in the test subjects pre-treatment.

For control groups, creams (or other topical formulations) containing all ingredients of the mastic isolated components formulations, except the mastic isolated components (for example, fatty acids, oils, stabilizers, emulsifiers, pigments, fragrances, etc.) are applied by subjects under identical conditions at identical times each day for identical total study time (i.e. ½-month, one-month, two-months, three-months, etc. up to six-months).

Similarly, control groups receiving just the cream (or other topical formulation) components, are also evaluated for degree of colonization and activity of related secondary conditions, and compared to the treatment groups.

### Example 15: Use of Mastic Extract, Essential Oil, and Isolated Components For In Vivo (Human) Treatment of Cell Hyperproliferation - Dandruff

Dilutions of mastic whole extract, acid extract, neutral extract, essential oil or isolated components are applied once or twice daily in a shampoo formulation containing about 1 to 25% extract, or about 1 to 10% isolated component, and optionally about 1 to 5% essential oil, to human test subjects (groups of 10 to 20 subjects per study for both control groups and those taking mastic compounds) exhibiting symptoms of cell hyperproliferation in the form of dandruff.

After treatment times varying from a range of a total of one-half month to three months or alternatively six months, the degree of cell hyperproliferation, in this case dandruff, is evaluated objectively (for example, by hair combining over collection trays for a specified number of combings, followed by determination of total mass of collected cells, microscopic evaluation and characterization of collected cells, etc) and subjectively (as reported by the test subject) relative to pre-treatment and to controls.

Control groups applying a shampoo lacking any mastic extract fraction or essential oil are also evaluated for degree of objective and subjective manifestations of cell hyperproliferation, in this case manifestations of dandruff, and compared to the treatment groups.

Also, the degree and the activity of secondary conditions such as dermatitis, scalp lesions, and the like which may be originally present in the test subjects, are evaluated and compared to similarly symptomatic subjects treated in similar kind with a placebo shampoo or a shampoo containing all ingredients as the mastic extract shampoo formulation except the mastic extract, and to the levels of such conditions in the test subjects pre-treatment. **Example 16** Use of Mastic Extracts, Essential Oil, and Isolated Components For *In Vivo*

### (Human) Treatment of Cell Hyperproliferation

Dilutions of mastic whole extract, acid extract, neutral extract, essential oil or isolated components are applied once to twice daily orally or topically, for example as a tablet, transdermal patch, cream or ointment, to human test subjects diagnosed with a hyperproliferative condition, such as a cancer, for example human epidermoid carcinoma, or pre-cancer, for example pre-cancerous skin growths known as actinic keratoses, to 20 subjects per study. Oral tablets may contain from about 5% to about 63% (w/w) whole extract; or from about 1% to about 25% (w/w) acid extract; or from about 5% to about 63% (w/w) neutral extract; or from about 1% to about 20% (w/w) isolated component; and optionally from about 0.25% to about 1.2% (w/w) of essential oil per tablet (see Example 9, Tables 12-15, above). Transdermal patches may contain about 1% to about 10% active mastic agent, depending upon the active component. Topical creams are formulated as above (see Example 9, Tables 16-17).

Topical treatments may include application once or twice daily, for total times varying from about one-half month up to six months, as needed. After such time, the degree of cell hyperproliferation is histopathologically evaluated according to standard protocols, relative to pre-treatment and to controls (as described above).

Oral treatments may include one to two tablets daily over the course of one-half month to up to 6 months, as required to reduce or control cell hyperproliferation.

### Additional References:

Dixon, M.F., R.M. Genta, J.H. Yardley and P. Correa. 1996. Classification and grading of gastritis. The updated Sydney System. Am. J. Surg. Pathol. 20:1161-81.
Lee, A., J. O'Rourke, M.C. De Ungria, B. Robertson, G. Daskalopoulos and M.F. Dixon. 1997. A standardized mouse model of Helicobacter pylori infection: introducing the Sydney strain. Gastroenterology. 112:1386-97.
Lu, J.J., C.L. Perng, R.Y. Shyu, C.H. Chen, Q. Lou, S.K Chong and C.H. Lee. 1999. Comparison of five PCR methods for detection of Helicobacter pylori DNA in gastric tissues. J Clin. Microbiol. 37:772-4.
Ilstrup, D.M. 1990. Statistical Methods in Microbiology. Clin Microbiol Rev 3:219-226.

## Claims

1. An oral or topical pharmaceutical formulation comprising a mastic extract product and a mastic essential oil, isolated from the species *Pistacia lentiscus* var. *chia*, for use in anti-microbial treatment of a subject.

2. A pharmaceutical formulation for use according to claim 1, wherein the anti-microbial treatment is against *H. Pylori, Propionibacterium, Staphylococcus* or *Pseudomonas*.

3. A pharmaceutical formulation for use according to claim 2, wherein the mastic extract product is a mastic whole extract product.

4. A pharmaceutical formulation for use according to claim 2, wherein the mastic extract product is a mastic acid extract product.

5. A pharmaceutical formulation for use according to claim 2, wherein the mastic extract product is a mastic neutral extract product.

## Patentansprüche

1. Orale oder topische pharmazeutische Formulierung, umfassend ein Extraktionsprodukt aus Mastix sowie ein essentielles Öl aus Mastix, isoliert aus der Spezies *Pistacia lentiscus* var. *chia*, zur antimikrobiellen Behandlung einer Person.

2. Pharmazeutische Formulierung zur Verwendung gemäß Anspruch 1, wobei die antimikrobielle Behandlung gegen *H. Pylori, Propionibacterium, Staphylococcus* oder *Pseudomonas* ist.

3. Pharmazeutische Formulierung zur Verwendung gemäß Anspruch 2, wobei das Extraktionsprodukt aus Mastix ein Vollextraktionsprodukt aus Mastix ist.

4. Pharmazeutische Formulierung zur Verwendung nach Anspruch 2, wobei das Extraktionsprodukt aus Mastix ein saures Extraktionsprodukt aus Mastix ist.

5. Pharmazeutische Formulierung zur Verwendung nach Anspruch 2, wobei das Extraktionsprodukt aus Mastix ein neutrales Extraktionsprodukt aus Mastix ist.

## Revendications

1. Formulation pharmaceutique orale ou topique comprenant un produit issu d'un extrait de mastic et une huile essentielle de mastic, et isolée de l'espèce *Pistacia lentiscus* var. *chia*, pour le traitement antimicrobe d'une personne.

2. Formulation pharmaceutique pour utilisation selon la revendication 1 dans laquelle le traitement antimicrobe est contre *H. Pylori, Propionibacterium, Staphylococcus* ou *Pseudomonas.*

3. Formulation pharmaceutique pour utilisation selon la revendication 2 dans laquelle le produit issu d'un extrait de mastic est un produit issu d'un extrait total de mastic.

4. Formulation pharmaceutique pour utilisation selon la revendication 2 dans laquelle le produit issu d'un extrait de mastic est un produit issu d'un extrait acide de mastic.

5. Formulation pharmaceutique pour utilisation selon la revendication 2 dans laquelle le produit issu d'un extrait de mastic est un produit issu d'un extrait neutre de mastic.
